# EUROPÄISCHE PATENTSCHRIFT

(11) **EP 3 238 620 B1**
(45) Veröffentlichungstag und Bekanntmachung des Hinweises auf die Patenterteilung: **20.12.2023**
(21) Anmeldenummer: 17167915.2
(22) Anmeldetag: 25.04.2017
(51) Int. Cl.: A61B 5/11, A63B 26/00, G06F 3/033, A61B 5/16, A63B 71/06, A63B 22/00

(54) **BEWEGUNGSERFASSUNGSPLATTE**
MOTION DETECTION PLATE
PLAQUE DE DÉTECTION DE MOUVEMENT

(30) Priorität: 25.04.2016 CH 5472016
(43) Veröffentlichungstag der Anmeldung: 01.11.2017
(73) Patentinhaber: Dividat AG, 8834 Schindellegi (CH)
(72) Erfinder: VAN HET REVE, Eva, 8834 Schindellegi (CH); AMIRTHAM, Adarsh John, 8852 Altendorf (CH); BADALLI, Bujar, 8805 Richterswil (CH); DE BRUIN, Eling D, 8418 Schlatt (CH)
(74) Vertreter: Rentsch Partner AG

(56) Entgegenhaltungen:
- EP-A1- 1 043 746
- WO-A1-2005/121935
- WO-A1-2016/049688
- JP-A- 2001 095 969
- JP-B2- 4 852 661
- US-A1- 2008 146 329
- US-A1- 2010 285 925
- US-A1- 2012 058 861
- US-B1- 7 645 211
- US-B1- 8 131 498

## Beschreibung

### GEBIET DER ERFINDUNG

Die vorliegende Erfindung betrifft eine Bewegungserfassungsplatte zur Erfassung von Bewegungen einer auf der Bewegungserfassungsplatte positionierten Person, eine Bewegungserfassungsvorrichtung und ein Verfahren zum Bereitstellen, dynamischen Erfassen und Verarbeiten von Bewegungsdaten.

### HINTERGRUND DER ERFINDUNG

Zur Förderung der Gesundheit und der körperlichen Aktivität existieren verschiedene Erfassungsvorrichtungen, welche insbesondere die Bewegungen der Personen, welche diese Erfassungsvorrichtungen benutzen, erfassen. Zu diesem Zweck von Beliebtheit sind in jüngerer Zeit zum Beispiel so genannte Fitness-Tracker, welche in Form von Armbändern am Handgelenk getragen werden können und verschiedene Werte des Trägers, wie die Schrittzahlen, Pulswerte etc. aufnehmen und abspeichern können.

Ein Verfahren zur Verbesserung der motorischen Fähigkeiten einer Person wird zum Beispiel in der WO2011/025518 beschrieben. Das Verfahren umfasst das Verwalten des Trainings von kognitiven Funktionen in einem Mass und in einer Art, welche wirksam ist, um die Mobilität des Subjekts zu verbessern. In einem ersten Schritt wird die motorische Funktion des Subjekts festgestellt, bevor das kognitive Training ausgeführt wird. Nach dem Ausführen des kognitiven Trainings wird die motorische Funktion des Subjekts wieder festgestellt und mit den Werten vor dem kognitiven Training verglichen. Beim Subjekt kann dabei getestet werden, ob eine Verbesserung z.B. der Gangparameter, Laufgeschwindigkeit, oder Verminderung der Fallhäufigkeit stattgefunden hat.

Eine Balancierplatte als Steuervorrichtung in einem Computersystem, das eine visuelle Displayvorrichtung umfasst, insbesondere in Verwendung in der Rehabilitation oder als Trainingsgerät wird in der WO201 5/130177 beschrieben. Die Balancierplatte umfasst eine Plattform und ein Bodenkontaktteil, um welches die Plattform schwenkbar angeordnet ist. Das Bodenkontaktteil weist eine gekrümmte, vielseitige Oberfläche auf. Die Oberfläche der Plattform enthält eine Ausnehmung, welche dazu geeignet ist, ein portables elektronisches Gerät mit einem Bewegungssensor aufzunehmen, das zur drahtlosen Übertragung von Bewegungsdaten zum Computersystem ausgebildet ist. Die Bewegungsdaten, welche durch Schwenken der Balancierplatte generiert werden, können vom Computersystem als Eingabeinformation genutzt werden. Der Bewegungssensor kann ein Gyroskop, ein Beschleunigungsmeter oder andere Sensoren umfassen. Die Plattform kann weiter Drucksensoren sowie Sensoren zur Messung der Schwenkung und der Beschleunigung umfassen.

Eine weitere Bewegungserfassungsvorrichtung entsprechend des Stands der Technik wird z.B. in JP4852661B2 beschrieben.

### DARSTELLUNG DER ERFINDUNG

Besonders bei Menschen, welche im Alltag aus verschiedenen Gründen nicht zu ausreichend Bewegung kommen, können Trainingssysteme zur Förderung der körperlichen und kognitiven Leistungsfähigkeit zum Erhalt und zur Verbesserung der Mobilität (in Form von sicherem Gehen und der Sturzprophylaxe bei Senioren) und zur Förderung der körperlichen Aktivität von Vorteil sein. Beispiele für Zielgruppen sind sind Menschen mit Beeinträchtigungen des Bewegungsapparats und/oder der perzeptuellen Fähigkeiten, Menschen höheren Alters und/oder Menschen nach Schlaganfällen, bei welchen vor allem die motorischen Fähigkeiten, die Wahrnehmung und die Mobilität eingeschränkt sind. Bei älteren Menschen oder Menschen nach Schlaganfällen sind die Fähigkeiten im Zusammenhang mit dem Gleichgewichtssinn und mit dem Gang entscheidende Faktoren, welche die Mobilität beeinträchtigen und bei welchen eine Verbesserung durch gezieltes Training und erhöhte Aktivität erwünscht ist.

Durch gezielten Einsatz von Trainingsprinzipien sollen die körperlichen und kognitiven Fähigkeiten verbessert werden.

Es ist daher eine Aufgabe der Erfindung, den Stand der Technik der Bewegungserfassung, insbesondere unter Nutzung von Bewegungserfassungsvorrichtungen für die Förderung der körperlichen Aktivität, zu verbessern.

Diese Aufgabe wird durch die Merkmale der unabhängigen Ansprüche gelöst. Bevorzugte Ausgestaltungen der Erfindung sind in den abhängigen Ansprüchen und in der vorliegenden Beschreibung und den Zeichnungen gegeben.

Die Erfindung betrifft eine Bewegungserfassungsplatte nach Anspruch 1.

In der Regel wird die Bewegungserfassungsplatte auf einer ebenen Oberfläche, z.B. auf dem Boden, positioniert. Auf die durch die Teilplatten ausgebildete Standfläche kann sich eine Person, welche die Bewegungserfassungsplatte nutzen möchte, positionieren und auf dieser Bewegungen ausführen, welche von der Bewegungserfassungsplatte erfasst werden. Die Bewegungserfassungsplatte ist durch die Unterteilung in Teilplatten mit jeweiligen Sensoren vorteilhafterweise dazu geeignet, ausgeführte Schritte der Person auf der Bewegungserfassungsplatte zu erfassen. Die unabhängigen Sensoren jeder Teilplatte bieten zudem den Vorteil, dass parallel zur Messung der Schritte auch Druckverteilungen aufgrund von Bewegungen der Person jeweils auf jeder Teilplatte erfasst werden können. Durch die Anordnung mit mindestens drei Sensoren auf einer Teilplatte können Druckverteilungen auf der Teilplatte lokal erfasst werden. Aus der Erfassung der Druckverteilungen auf den Teilplatten kann die Druckverteilung auf der Bewegungserfassungsplatte ermittelt werden. Die Sensoren können Druckverteilungen mit Vorteil dynamisch messen, d.h. Druckveränderungen vorzugsweise in Echtzeit erfassen.

Optional ist über den Teilplatten eine Folie, vorzugsweise aus Kunststoff, angeordnet, so dass Zwischenräume zwischen den Teilplatten durch die Folie überdeckt werden. Die Folie kann transparent oder semitransparent sein. Vorzugsweise ist die Folie aus einem rutschfesten Material ausgebildet. Die Folie bietet den Vorteil, dass eine einheitliche Standfläche ohne Zwischenräume gebildet werden kann.

Die Sensoren umfassen Kraftsensoren. Bevorzugt weisen die Kraftsensoren eine maximale statische Belastbarkeit von je 50 kg auf. Die Kraftsensoren sind derart ausgebildet, dass die Teilplatten in Bezug auf Gewichtsänderungen eine Empfindlichkeit von 1 kg aufweisen. Das Ansteuern der Sensoren durch die Kontrollvorrichtung kann insbesondere das Einstellen und/oder das Auslesen der Sensoren umfassen. Optional kann die Kontrollvorrichtung einzelne Sensoren aktivieren oder deaktivieren.

Vorzugsweise ist die Kontrollvorrichtung unterhalb der Teilplatten angeordnet, so dass die Kontrollvorrichtung von aussen nicht sichtbar ist. Ausserdem bietet eine solche Anordnung den Vorteil, dass die Kontrollvorrichtung vor Beschädigungen geschützt angeordnet ist.

Die Bewegungserfassungsplatte bietet den Vorteil, dass die auf ihr positionierte Person durch die Rückmeldungsvorrichtung eine Rückmeldung über die ausgeführten Bewegungen erhalten kann und somit das motorische Lernen gezielt gefördert wird. Die Rückmeldung über die ausgeführten Bewegungen kann eine direkte Rückmeldung sein ("direct feedback on performance"). Die Ausgabe von taktilen und/oder haptischen Rückmeldungen ist von Vorteil, falls die betreffende Person über eingeschränkte Seh- und/oder Hörfähigkeiten verfügt, was bei älteren Menschen oft der Fall sein kann. Vorzugsweise wird die Rückmeldungsvorrichtung durch die Kontrollvorrichtung angesteuert. Die Kontrollvorrichtung kann die Stärke und/oder den Zeitpunkt und/oder die Dauer der Rückmeldung steuern.

Die Rückmeldungsvorrichtung kann auch dazu dienen, visuelle und/oder auditive und/oder taktile Bewegungsanweisungen auszugeben. Auditive Bewegungsanweisungen können zum Beispiel durch an der Bewegungserfassungsplatte angeordnete Lautsprecher erfolgen. Visuelle Bewegungsanweisungen können durch Anzeigevorrichtungen erfolgen. Taktile Bewegungsanweisungen können durch an der Bewegungserfassungsplatte angeordnete Antriebselemente erfolgen. Die auditiven, visuellen oder taktilen Bewegungsanweisungen können alternativ oder in Ergänzung zueinander verwendet werden, um die auf der Bewegungserfassungsplatte positionierte Person für bestimmte Bewegungen anzuweisen.

In einer bevorzugten Ausgestaltung umfasst die Rückmeldungsvorrichtung Vibrationsmotoren zur Erzeugung einer haptischen oder taktilen Rückmeldung, wobei vorzugsweise an jeder Teilplatte jeweils mindestens ein durch die Kontrollvorrichtung ansteuerbarer Vibrationsmotor angeordnet ist.

Bevorzugt sind die Vibrationsmotoren jeweils derart unter den Teilplatten angeordnet, dass die Teilplatten eine taktile Rückmeldung ausgeben können. Alternativ oder in Ergänzung können die Vibrationsmotoren unter Nutzung der Sensoren derart ausgebildet sein, dass eine haptische Rückmeldung ausgegeben werden kann. Vorzugsweise sind die Vibrationsmotoren bezüglich der Teilplatten mittig angeordnet. Die Kontrollvorrichtung kann die Vibrationsmotoren derart ansteuern, dass die Stärke und/oder der zeitliche Verlauf der Vibration variiert werden kann. Zum Beispiel kann durch die Kontrollvorrichtung ein Vibrationsmotor derart eingestellt werden, dass der Vibrationsmotor einen Rechteckpuls oder einen allmählich ansteigenden bzw. abfallenden Puls fahren kann. Die Kontrollvorrichtung kann mit Vorteil auch die Frequenz der Vibration einstellen. Optional kann die Kontrollvorrichtung einzelne Vibrationsmotoren aktivieren oder deaktivieren.

In einer bevorzugten Ausgestaltung umfasst die Rückmeldungsvorrichtung ausleuchtbare Anzeigevorrichtungen, welche an der Bewegungserfassungsplatte angeordnet sind und durch die Kontrollvorrichtung ansteuerbar sind.

Vorzugsweise sind an jeder Teilplatte unabhängig ansteuerbare Anzeigevorrichtungen angeordnet. Durch das Aktivieren einer Anzeigevorrichtung auf einer Teilplatte kann zum Beispiel die auf der Bewegungserfassungsplatte positionierte Person dazu angewiesen werden, einen Schritt auf diese Teilplatte auszuführen oder das Gewicht auf diese Teilplatte zu verlagern. Alternativ oder in Ergänzung kann durch das Aktivieren einer Anzeigevorrichtung auf einer Teilplatte eine visuelle Rückmeldung über eine ausgeführte Bewegung, z.B. ob diese einer vorgegebenen Bewegungsanweisung entspricht oder von ihr zu einem bestimmten Grad abweicht, ausgegeben werden.

In einer Ausgestaltung umfassen die Anzeigevorrichtungen Pfeile, so dass Richtungsangaben anzeigbar sind.

In einer bevorzugten Ausgestaltung umfassen die Anzeigevorrichtungen mindestens teilweise transparente Flächen auf der Bewegungserfassungsplatte. Die teilweise transparenten Flächen können in einer Ausgestaltung durch in die Bewegungserfassungsplatte integrierte nichttransparente Schablonen mit Öffnungen ausgebildet sein. Vorzugsweise umfassen die Anzeigevorrichtungen weiter Leuchtmittel, welche unterhalb der Bewegungserfassungsplatte angeordnet sind und die teilweise transparenten Flächen ausleuchten können. Die Leuchtmittel umfassen in einer Ausgestaltung LEDs. Die Leuchtmittel können mit Vorteil durch die Kontrollvorrichtung angesteuert werden. Die Anzeigevorrichtungen können transparente oder lichtstreuende Folien umfassen. Die Folien sind bevorzugt aus Kunststoff hergestellt. Alternativ oder in Ergänzung können die Anzeigevorrichtungen satiniertes Glas umfassen. In einer Ausgestaltung umfassen die Anzeigevorrichtungen Einscheiben-Sicherheits-Glas (ESG). In einer Variante umfasst die Anzeigevorrichtung Verbundssicherheitsglas (VSG). Die Bestandteile der Anzeigevorrichtungen können derart ausgewählt und/oder angeordnet sein, dass eine flächige oder punktförmige Ausleuchtung der Anzeigevorrichtungen bereitgestellt wird. Optional können die Anzeigevorrichtungen Spiegel umfassen.

In einer Ausgestaltung sind die Anzeigevorrichtungen ausgebildet, Anzeigen verschiedenfarbig auszugeben. Zum Beispiel kann eine erste Farbe zur Anzeige von auszuführenden Bewegungen benutzt werden und eine zweite Farbe zur Anzeige von ausgeführten Bewegungen, welche Anweisungen entsprechend korrekt ausgeführt wurden. Eine dritte Farbe kann zum Beispiel benutzt werden, um eine Anweisungen nicht entsprechende falsche Bewegung anzuzeigen. Optional kann die Helligkeit der Anzeigen variiert werden, wobei dies bevorzugt durch die Kontrollvorrichtung gesteuert wird.

In einer Ausgestaltung umfasst die Bewegungserfassungsplatte Leuchtmittel, welche zur Ausleuchtung von Trennlinien zwischen den Teilplatten dienen. Dies bietet den Vorteil, dass die Teilplatten visuell besser voneinander abgetrennt werden können und z.B. die Orientierung für die auf der Bewegungserfassungsplatte positionierten Person erleichtert.

In einer Ausgestaltung sind in der Kontrollvorrichtung Kalibrierwerte für die Sensoren und/oder die Rückmeldungsvorrichtung speicherbar.

Die Kontrollvorrichtung bietet daher den Vorteil, dass die Sensoren und/oder die Rückmeldungsvorrichtung zentral kalibriert werden können.

Vorzugsweise umfasst die Kontrollvorrichtung einen Datenspeicher, auf welchem die Kalibrierwerte speicherbar sind.

In einer Ausgestaltung umfasst die Bewegungserfassungsplatte eine Kommunikationsschnittstelle zur Ausgabe von durch die Sensoren gemessenen Werten.

Die Kommunikationsschnittstelle bietet den Vorteil, dass die von den Sensoren erfassten Werte extern weitergeleitet und ausgewertet werden können. Weiter bietet die Kommunikationsschnittstelle den Vorteil, dass die Bewegungserfassungsplatte an eine externe Kontrollvorrichtung, z.B. einen PC oder ein mobiles Gerät, angeschlossen werden kann. Die externe Kontrollvorrichtung kann über eine Anwendersoftware oder eine Firmware die Bewegungserfassungsplatte einstellen, z.B. Kalibrierwerte einspeisen, und/oder Messdaten aus den Sensoren auslesen und bearbeiten, und/oder die Bestandteile der Bewegungserfassungsplatte, wie z.B. die Rückmeldungsvorrichtung, steuern. Die über die Kommunikationsschnittstelle ausgelesenen Messdaten können optional in eine Datenbank geladen werden, von welcher sich z.B. ein Trainingsmanager oder die trainierende Person selbst die Messdaten herunterladen kann. Die Kommunikationsschnittstelle kann verschiedene Anschlüsse umfassen, wie z.B. RJ45-Ethernet Anschlüsse, serielle Schnittstellen, Bluetooth-Module oder steckbare Wifi-Module.

In einer bevorzugten Ausgestaltung weist die Bewegungserfassungsplatte eine Bodenplatte auf, über welche die Teilplatten angeordnet sind, wobei die Sensoren zwischen der Bodenplatte und den Teilplatten angeordnet sind.

Die Bodenplatte ist mit Vorteil mit einer ausreichenden Steifigkeit derart ausgestattet, dass kleine Unebenheiten im Boden, z.B. aufgrund eines weichen Bodens, kompensiert werden können. Die Bodenplatte bietet zudem den Vorteil, dass die Stabilität der Bewegungserfassungsplatte erhöht werden kann. Bevorzugt ist die Bodenplatte derart unter den Teilplatten angeordnet, dass zwischen den Teilplatten und der Bodenplatte ein Zwischenraum gebildet wird. In diesem Zwischenraum können verschiedene Bestandteile der Bewegungserfassungsplatte angeordnet sein. Zum Beispiel können die Sensoren und/oder die Kontrollvorrichtung und/oder die Leuchtmittel der Anzeigevorrichtungen und/oder die Vibrationsmotoren in diesem Zwischenraum angeordnet sein. Die Bodenplatte bietet den weiteren Vorteil, dass eine stabile, feste Oberfläche bereitgestellt wird, auf welcher Bestandteile der Bewegungserfassungsplatte, welche möglichst ruhend positioniert sein sollten, wie z.B. die Kontrollvorrichtung oder die Leuchtmittel, angeordnet werden können. Weiter bietet die Anordnung mit der Bodenplatte den Vorteil, dass die im Zwischenraum angeordneten Bestandteile einerseits von aussen nicht sichtbar sind und andererseits durch die Teilplatten sowie die Bodenplatte geschützt sind. Ausserdem können Anschlüsse und Kabel für Bestandteile der Bodenplatte im Zwischenraum verstaut werden.

In einer bevorzugten Ausgestaltung umfasst die Bewegungserfassungsplatte fünf Teilplatten, wobei eine Teilplatte als zentral angeordnete Hauptteilplatte ausgebildet ist und vier Teilplatten als Seitenteilplatten um die zentrale Hauptteilplatte herum angeordnet sind.

Die zentrale Hauptteilplatte dient mit Vorteil als zentrale Standfläche, auf welche sich eine Person z.B. zum Trainingsbeginn stellen kann. Von dieser Position aus kann die Person dann Schritte in vier Richtungen auf die jeweiligen Seitenteilplatten ausführen, wobei die auszuführenden Schritte vorzugsweise mittels einer Stimulationsvorrichtung, z.B. mittels Anzeigen der Seitenteilplatten oder einem Display, angezeigt werden. Zum Abschluss einer Trainingssession kann die Person z.B. angewiesen werden, auf die Hauptteilplatte zurückzutreten, so dass ein Abschluss der Trainingssession von der Kontrollvorrichtung registriert und die in dieser Trainingssession von den Sensoren ermittelten Bewegungswerte über die Kommunikationsschnittstelle gruppiert ausgegeben werden können. Bevorzugt ist die zentrale Hauptteilplatte rechteckig, vorzugsweise quadratisch, ausgebildet. Die vier Seitenteilplatten sind vorzugsweise trapezförmig ausgebildet und derart um die Hauptteilplatte herum angeordnet, dass die Hauptteilplatte und die Seitenteilplatten zusammen eine rechteckige, vorzugsweise quadratische, Standfläche ausbilden. Optional sind die am Rand der Bewegungserfassungsplatte liegenden Ränder von drei Seitenteilplatten mit einem Gefälle abgeschrägt ausgebildet. Der Rand der Seitenteilplatte ohne Gefälle am Rand der Bewegungserfassungsplatte kann dabei als Anschlussfläche für Steckverbindungen dienen. Optional können Teilflächen der abgeschrägten Ränder der Seitenteilplatten ausleuchtbar ausgebildet sein.

In einer Variante weist die Bewegungserfassungsplatte einen Rahmen auf, der umlaufend um die Teilplatten herum angeordnet ist. Optional ist der Rahmen mit einem Gefälle abgeschrägt ausgebildet.

In einer Ausgestaltung sind an jeder Teilplatte vier Sensoren peripher angeordnet.

Die Anordnung mit vier Sensoren pro Teilplatte bietet den Vorteil, dass die Druckverteilungen auf den Teilplatten eindeutig ermittelt und Artefakte bei der Korrelation der Druckmesswerte vermieden werden können.

In einer Ausgestaltung umfasst die Bewegungserfassungsplatte einen Rahmen, an welchen eine Griffvorrichtung lösbar anbringbar ist.

Die Griffvorrichtung bietet den Vorteil, dass sich eine Person, welche die Bewegungserfassungsplatte nutzen möchte, daran festhalten kann. Dies kann insbesondere für ältere Personen mit unsicherem Stand von Vorteil sein. Die Griffvorrichtung kann in einer Ausgestaltung in Form eines Handlaufs ausgebildet sein.

Die Erfindung betrifft weiter eine Bewegungserfassungsvorrichtung nach Anspruch 8.

Neben der Erfassung von Bewegungen der auf der Bewegungserfassungsplatte positionierten Person dient die Bewegungserfassungsvorrichtung vorteilhafterweise dazu, die betreffende Person zu Bewegungen, vorzugsweise im Rahmen eines Trainingsprogramms, zu animieren. Zu diesem Zweck kann die Stimulationsvorrichtung Bewegungsanweisungen ausgeben, nach welchen die Person Bewegungen auf der Bewegungserfassungsplatte ausführen kann. Die Bewegungsanweisungen umfassen in der Regel Richtungsangaben, nach welchen die Person Schritte in die betreffende Richtung ausführen kann. Die Bewegungsanweisungen können aber zu den Richtungsangaben auch Anweisungen zur Gewichtsverlagerung ausgeben, so dass die Person ihr Gewicht in die entsprechende Richtung verlagern kann. Die nach den Bewegungsanweisungen ausgeführten Bewegungen der Person können von den Sensoren der Bewegungserfassungsplatte erfasst werden. Die von den Sensoren erfassten Bewegungen können mit den entsprechenden Bewegungsanweisungen verglichen werden, um z.B. den Erfolg der Person in einem bestimmten Trainingsprogramm zu bewerten und weitere Etappen im Trainingsprogramm vorzusehen.

Die Stimulationsvorrichtung umfasst zur Ausgabe von visuellen Bewegungsanweisungen vorzugsweise ein Display, z.B. eines Computers oder eines mobilen Geräts, zu welchem die Person auf der Bewegungserfassungsplatte Sichtkontakt hat.

Alternativ oder in Ergänzung können die Bewegungsanweisungen der Stimulationsvorrichtung indirekte Anweisungen in Form von Reizen oder Stimuli umfassen, welche nicht zwingend als direkte Bewegungsanweisungen erkennbar sind. Optional können die Reize oder Stimuli komplexe Signale umfassen, welche erst nach einer kognitiven Verarbeitung als konkrete Bewegungsanweisungen erkennbar werden.

In einer Ausgestaltung wird die Stimulationsvorrichtung über die Kontrollvorrichtung angesteuert. Dies hat den Vorteil, dass die Steuerung der Sensoren der Bewegungserfassungsplatte, der Rückmeldungsvorrichtung und der Stimulationsvorrichtung zentral über die Kontrollvorrichtung vorgenommen werden kann.

Auditive Bewegungsanweisungen können zum Beispiel durch Lautsprecher erfolgen. Die auditiven und visuellen Bewegungsanweisungen können alternativ oder in Ergänzung zueinander verwendet werden, um die auf der Bewegungserfassungsplatte positionierte Person zu bestimmten Bewegungen zu animieren.

In einer Ausgestaltung ist die Stimulationsvorrichtung derart mit der Rückmeldungsvorrichtung gekoppelt, dass den visuellen und/oder auditiven Bewegungsanweisungen der Stimulationsvorrichtung entsprechende taktile und/oder haptische und/oder visuelle und/oder auditive Rückmeldungen bzw. Bewegungsanweisungen über die Rückmeldungsvorrichtung ausgegeben werden können.

In einer Ausgestaltung umfasst die Bewegungserfassungsvorrichtung eine Identifikationsvorrichtung mit einem RFID-Reader oder Barcode-Reader.

Mittels der Identifikationsvorrichtung kann eine Person z.B. mit einer Identifikationskarte oder einem Identifikationsband, welche einen RFID-Chip oder einen Barcode aufweist, sich bei einer Trainingssession identifizieren. Dies bietet den Vorteil, dass die nach der Identifikation erfassten Werte der Bewegungserfassungsplatte eindeutig einer Person zugeordnet werden können. Entsprechend können der Person zugeordnete Bewegungsanweisungen über die Stimulationsvorrichtung ausgegeben werden.

In einer Ausgestaltung umfasst die Bewegungserfassungsvorrichtung eine mit der Bewegungserfassungsplatte lösbar verbindbare Griffvorrichtung, vorzugsweise in Form eines Handlaufs.

Die Griffvorrichtung bietet den Vorteil, dass sich eine Person, welche die Bewegungserfassungsplatte nutzen möchte, sich daran festhalten kann. Dies kann insbesondere für ältere Personen mit unsicherem Stand von Vorteil sein.

In einer Ausgestaltung ist die Identifikationsvorrichtung an der Griffvorrichtung angeordnet.

Optional umfasst die Griffvorrichtung Ablagevorrichtungen zur Ablage von Utensilien der trainierenden Person, wie z.B. ein Handtuch oder Armbanduhren etc.

In einer Ausgestaltung umfasst die Griffvorrichtung Antriebselemente, welche eine haptische Rückmeldung bereitstellen können. Eine trainierende Person kann in dieser Ausgestaltung über die Griffvorrichtung eine haptische Rückmeldung zu den von ihr ausgeführten Bewegungen erhalten. Alternativ oder in Ergänzung kann die Griffvorrichtung eine Steuervorrichtung aufweisen, welche die trainierende Person für bestimmte Eingaben an die Bewegungserfassungsvorrichtung verwenden kann. Die haptische Rückmeldung kann z.B. in Reaktion auf eine Eingabe über die Steuervorrichtung der Griffvorrichtung ausgegeben werden.

Die Erfindung betrifft weiter ein Verfahren nach Anspruch 10.

In der Regel wird das erfindungsgemässe Verfahren in einer Vielzahl von Zyklen durchgeführt. Ein Zyklus beginnt mit dem Bereitstellen eines Satzes von Stimulationssignalen mit einer vorgegebenen Komplexität und endet mit dem Bereitstellen eines neuen Satzes von Stimulationssignalen mit veränderter Komplexität. Mit diesem neuen Satz von Stimulationssignalen kann wiederum ein neuer Zyklus begonnen werden.

Der Begriff der Komplexität der Stimulationssignale, der Komplexität der primären Bewegungsdaten und der Differenzdaten ist dem Fachmann bekannt. Insbesondere ist darunter der Ausmass der Komplexität in der zeitlichen Abfolge der in den Stimulationssignalen, bzw. primären Bewegungsdaten, zu stimulierenden bzw. kodierten Bewegungsvorgänge und/oder die Anzahl und/oder die Art der zu stimulierenden bzw. kodierten Bewegungsvorgänge zu verstehen.

Vorzugsweise umfasst die Bewegungserfassungsvorrichtung eine Datenbank, in welche Sätze von Stimulationssignalen mit vorgegebenen Komplexitäten abgelegt sein können und von welcher sie bereitgestellt werden können.

In einer Ausgestaltung werden vor dem Bereitstellen der Stimulationssignale von einer Identifikationsvorrichtung Daten zur Person erfasst, anhand welcher zu dieser Person (bzw. ihrer Fitness oder Mobilitätsmöglichkeiten) passende Stimulationssignale ausgewählt und bereitgestellt werden. Alternativ oder in Ergänzung können nach der Identifikation der Person an der Identifikationsvorrichtung Daten der letzten Trainingssession aufgerufen werden und anhand dieser Daten Stimulationssignale bereitgestellt werden.

Die primären Bewegungsdaten dienen dazu, die bereitgestellten Stimulationssignale unverändert abzubilden, so dass diese weiterverwendet oder über die hergeleiteten primären Bewegungsdaten mit sekundären Bewegungsdaten aus dem Verarbeitungskanal verglichen werden können.

Optional kann ein Zwischenspeicher vorgesehen sein, in welchen eine Kopie des bereitgestellten Satzes der Stimulationssignale bzw. primären Bewegungsdaten abgelegt wird.

Die Störstelle im Verarbeitungskanal dient dazu, von den in den Verarbeitungskanal eingespiesenen Stimulationssignalen sekundäre Bewegungsdaten herzuleiten. Herleiten ist in diesem Zusammenhang breit zu verstehen und kann verschiedene Vorgänge beinhalten, unter Anderem z.B. fehlerhaftes Abbilden der Stimulationssignale, oder z.B. Erzeugen von sekundären Bewegungsdaten, wobei die Stimulationssignale als Vorgabe zur Erzeugung der sekundären Bewegungsdaten dienen, etc.

Aus dem Vergleich der primären Bewegungsdaten mit den sekundären Bewegungsdaten aus dem Verarbeitungskanal können Abweichungen der Bewegungsdaten voneinander ermittelt werden und diese Abweichungen in Differenzdaten kodiert werden.

Die Komplexität des Satzes der Stimulationssignale wird als Funktion der Differenzdaten verändert. Vorzugsweise wird die Komplexität umgekehrt proportional zu den in den Differenzdaten kodierten Abweichungen verändert. Das Ausmass der Abweichungen kann bevorzugt in Bezug auf einen in der vorgegebenen Komplexität definierten Abweichungs-Toleranzwert ermittelt werden.

Das erfindungsgemässe Verfahren bietet den Vorteil, dass die Komplexität der Stimulationssignale durch die Vielzahl an Zyklen an die Störstelle, bzw. an die in der Störstelle erwirkten Herleitungen der sekundären Bewegungsdaten, angepasst werden kann. Vorzugsweise nimmt das Ausmass der Veränderung der Komplexität anhand der Differenzdaten mit jedem Zyklus ab.

In einer Ausgestaltung ist es vorgesehen, unter einem vorgegebenen Schwellenwert die Komplexität der Stimulationssignale mit jedem Zyklus zu erhöhen, falls die Abweichung der sekundären Bewegungsdaten von den primären Bewegungsdaten unter diesem Schwellenwert liegt.

Eine Erhöhung der Komplexität der Stimulationssignale für Abweichungen unter einem Schwellenwert, vorzugsweise unter dem definierten Abweichungs-Toleranzwert, kann vorteilhaft sein für eine Störstelle, welche lernfähig ist, d.h. welche z.B. mit jedem Zyklus bis zu einem gewissen Ausmass die Abweichungen zwischen primären und sekundären Bewegungsdaten reduzieren kann.

Das Verfahren umfasst das Herleiten der sekundären Bewegungsdaten in der Störstelle folgende Schritte:
a) Erzeugen von räumlichen und/oder zeitlichen Bewegungsanweisungen mittels einer Stimulationsvorrichtung anhand der Stimulationssignale;
b) Erfassen von sekundären Bewegungsdaten mittels einer Bewegungserfassungsplatte gemäss der vorliegenden Beschreibung, wobei die sekundären Bewegungsdaten durch Bewegungen einer auf der Bewegungserfassungsplatte positionierten Person gemäss den Bewegungsanweisungen erzeugt werden.

Vorzugsweise umfasst die Stimulationsvorrichtung ein Display, z.B. eines Computers oder tragbaren Gerätes, auf welcher die Bewegungsanweisungen ausgegeben werden. Die Bewegungsanweisungen werden z.B. durch den Computer, der die Stimulationssignale von einer Datenbank lädt, erzeugt. Vorzugsweise hat sich die Person mittels einer Identifikationsvorrichtung identifiziert und der Computer anhand der Identifikationsdaten zur Person einen für diese geeigneten Satz an Stimulationssignalen von der Datenbank geladen. Die Person auf der Bewegungserfassungsplatte kann nun sekundäre Bewegungsdaten erzeugen, indem sie gemäss den Bewegungsanweisungen auf der Stimulationsvorrichtung Bewegungen auf der Bewegungserfassungsplatte ausführt. Je nach Komplexität der Stimulationssignale, nach welchen die Bewegungsanweisungen erzeugt wurden, können die von der Person erzeugten sekundären Bewegungsdaten von den primären Bewegungsdaten stärker oder schwächer abweichen. Es besteht auch hier der Vorteil, dass die Komplexität der Stimulationssignale, bzw. der primären Bewegungsdaten und somit der Bewegungsanweisungen dynamisch an die auf der Bewegungserfassungsplatte positionierte Person und die von ihr erzeugten sekundären Bewegungsdaten angepasst werden kann. Vorzugsweise wird die Komplexität der Stimulationssignale mit jedem Zyklus so weit verändert, bis die Abweichungen der von der Person erzeugten sekundären Bewegungsdaten von den primären Bewegungsdaten unter einem Schwellenwert, vorzugsweise einem Abweichungs-Toleranzwert, zu liegen kommen. Unter diesem Schwellenwert kann mit Vorteil die Komplexität der Stimulationssignale mit jedem Zyklus wieder erhöht werden, so dass die auf der Bewegungserfassungsplatte positionierte Person zu komplexeren Bewegungen animiert werden kann, so weit, bis die Abweichungen wieder zu gross werden und die Komplexität wieder angepasst werden kann.

In einer Ausgestaltung des Verfahrens wird nach der Erzeugung einer Bewegungsanweisung ein Zeitfenster definiert, wobei ein erstes in diesem Zeitfenster erfasstes sekundäres Bewegungsdatenelement einem der Bewegungsanweisung zugehörigen Stimulationssignal zugeordnet wird und die Zeit zwischen Anzeigen der Bewegungsanweisung und Erfassen des sekundären Bewegungsdatenelements als Reaktionszeitparameter in die Bildung eines Differenzdatenelements aufgenommen wird.

Der Reaktionszeitparameter bietet vorteilhafterweise einen weiteren Parameter zur Bildung der Differenzdaten, was sich auf die Veränderung der Komplexität der Stimulationssignale auswirkt. Dies bietet den Vorteil, dass die Komplexität der Stimulationssignale noch genauer auf die Möglichkeiten einer auf der Bewegungserfassungsplatte positionierten Person angepasst werden kann.

In einer Ausgestaltung des Verfahrens werden die sekundären Bewegungsdaten aus den von den Sensoren der Bewegungserfassungsplatte erfassten Druckverteilungswerten ermittelt.

Die Rohwerte der Sensoren können zum Beispiel von der Kontrollvorrichtung ausgelesen werden und über einen Analog-Digital-Wandler in digitale sekundäre Bewegungsdaten umgewandelt werden. Über die Kommunikationsschnittstelle können die sekundären Bewegungsdaten an ein Verarbeitungsmodul, z.B. an einen Computer, ausgegeben werden, auf welchem das Vergleichen mit den primären Bewegungsdaten durchgeführt werden kann.

In einer bevorzugten Ausgestaltung des Verfahrens wird bei oder ab einer vorgegebenen Differenz zwischen den sekundären Bewegungsdaten und den anhand der Stimulationssignale erzeugten Bewegungsanweisungen eine auditive und/oder visuelle und/oder haptische oder taktile Sofortrückmeldung durch die Rückmeldungsvorrichtung ausgegeben. Eine solche Sofortrückmeldung wird bevorzugt als eine negative Sofortrückmeldung bezeichnet.

Die negative Sofortrückmeldung bietet den Vorteil, dass eine Person auf der Bewegungserfassungsplatte bei einer Bewegung, welche sekundäre Bewegungsdaten erzeugt hat, welche in einem Ausmass grösser gleich der vorgegebenen Differenz von den Bewegungsanweisungen abweicht, eine sofortige Rückmeldung erhält.

Alternativ oder in Ergänzung wird bis zu einer vorgegebenen Differenz zwischen den sekundären Bewegungsdaten und den anhand der Stimulationssignale erzeugten Bewegungsanweisungen eine auditive und/oder visuelle und/oder haptische oder taktile (positive) Sofortrückmeldung durch die Rückmeldungsvorrichtung ausgegeben. Die positive Sofortrückmeldung bietet den zur negativen Sofortrückmeldung analogen Vorteil, wobei die sofortige Rückmeldung bei einer Abweichung kleiner gleich der vorgegebenen Differenz erhalten werden kann.

Es ist einer ist ein Korrekturzeitfenster vorgesehen, innerhalb welchem die Person nach einer Sofortrückmeldung gegebenenfalls eine zu stark von den Bewegungsanweisungen abweichende Bewegung, z.B. einen Schritt in die falsche Richtung, korrigieren bzw. noch einmal auf den Bewegungsanweisungen entsprechende Weise ausführen kann. Gelingt die Korrektur innerhalb des Korrekturzeitfensters, kann z.B. vorgesehen werden, dass das ursprüngliche abweichende sekundäre Bewegungsdatenelement gelöscht und durch das korrigierte sekundäre Bewegungsdatenelement ersetzt wird.

In einer Ausgestaltung des Verfahrens werden die primären und/oder sekundären Bewegungsdaten und/oder Differenzdaten und/oder Stimulationssignale in einer Datenbank gespeichert, wobei die Datenbank vorzugsweise mit einer Managementoberfläche zur Bereitstellung eines Zugriffs auf die Datenbank verbunden ist. Die Managementoberfläche bietet den Vorteil, dass z.B. durch eine trainingsleitende Person auf die Bewegungsdaten und/oder Stimulationssignale zugegriffen werden kann. Weiter vorteilhaft kann ein Trainingsfortschritt über die Managementoberfläche überwacht werden. Vorzugsweise können neue Sätze an Stimulationssignale über die Managementoberfläche auf die Datenbank abgelegt werden. Auf die Managementoberfläche kann bevorzugt über einen Computer oder ein tragbares Gerät etc. zugegriffen werden.

### LISTE DER FIGUREN

Ausführungsformen werden anhand der nachfolgenden schematischen Figuren und der dazugehörigen Beschreibungen näher erläutert. Es zeigen:
- Fig. 1: eine perspektivische Ansicht einer Ausführungsform einer Bewegungserfassungsplatte;
- Fig. 2: eine Ansicht der Bewegungserfassungsplatte gemäss Fig. 1 von oben;
- Fig. 3: eine Schnittansicht der Bewegungserfassungsplatte gemäss Fig. 1 entlang der Linie A-A;
- Fig. 4: eine perspektivische Ansicht einer Ausführungsform einer Bewegungserfassungsplatte mit einer Griffvorrichtung;
- Fig. 5: eine schematische Darstellung eines Ausführungsbeispiels des Verfahrens.

### BESCHREIBUNG EXEMPLARISCHER AUSFÜHRUNGSFORMEN

Um die Erfindung zu veranschaulichen, werden bevorzugte Ausführungsformen mit Bezug auf die Figuren näher beschrieben.

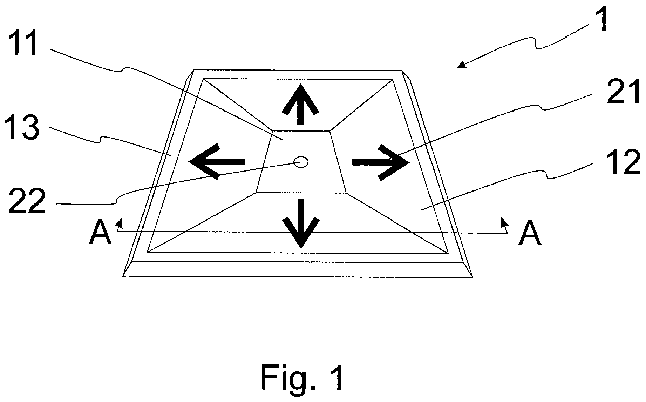

Figur 1 zeigt eine perspektivische Ansicht einer Ausführungsform einer Bewegungserfassungsplatte 1. Die Bewegungserfassungsplatte 1 umfasst eine Hauptteilplatte 11 und vier Seitenteilplatten 12. Die Hauptteilplatte 11 ist quadratisch und die Seitenteilplatten 12 trapezförmig ausgebildet. Die Hauptteilplatte 11 und die Seitenteilplatten 12 sind derart aneinander anliegend angeordnet, dass eine gemeinsame Standfläche ausgebildet wird. Die Bewegungserfassungsplatte 1 weist weiter einen Rahmen 13 auf, der um die Seitenteilplatten 12 umlaufend angeordnet ist. Die Seitenteilplatten 12 weisen als pfeilförmige Anzeigevorrichtungen 21 auf, welche eine visuelle Rückmeldung ausgeben können. Die pfeilförmigen Anzeigenvorrichtungen 21 sind ausleuchtbar ausgebildet. Die Hauptteilplatte 11 weist eine kreisförmige Anzeigevorrichtung 22 auf, welche ebenfalls ausleuchtbar ist.

Die Anzeigevorrichtungen 21, 22 dienen dazu, eine auf der Bewegungserfassungsplatte 1 positionierte Person zu Bewegungen zu animieren und/oder Rückmeldung über ausgeführte Bewegungen auszugeben.

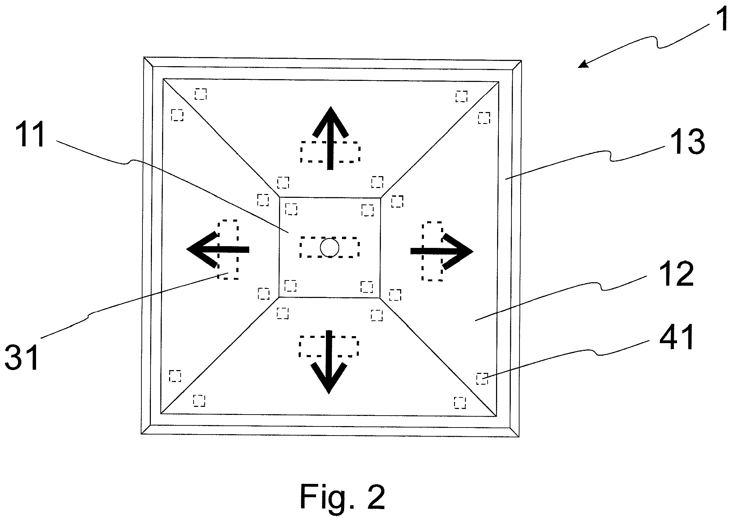

Figur 2 zeigt eine Ansicht der Bewegungserfassungsplatte 1 aus Fig. 1 von oben. Bestandteile der Bewegungserfassungsplatte 1, welche sich unterhalb der Hauptteilplatte 11 sowie der Seitenteilplatten 12 befinden, sind gestrichelt angedeutet. Unterhalb jeder Seitenteilplatte 12 befinden sich peripher angeordnete Kraftsensoren 41, welche Druckverteilungen auf jeder Seitenteilplatte 12 erfassen können. Weiter befindet sich unter jeder Seitenteilplatte 12 ein mittig angeordneter Vibrationsmotor 31, mittels welcher die Seitenteilplatten 12 taktile und/oder haptische Rückmeldung ausgeben können. Unterhalb der Hauptteilplatte 11 sind an den Ecken ebenfalls Kraftsensoren und zentral ein Vibrationsmotor angeordnet.

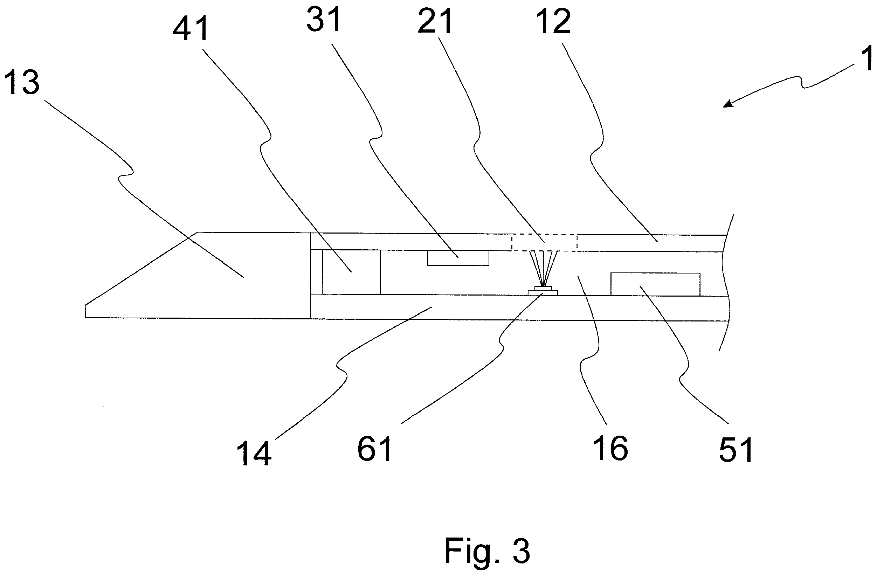

Figur 3 zeigt eine Schnittansicht der Bewegungserfassungsplatte aus Figur 1 entlang der Linie A-A. Der Rahmen 13 ist mit einem Gefälle abgeschrägt ausgebildet, was beim Betreten und Verlassen der Bewegungserfassungsplatte 1 Verletzungsgefahren reduziert. Die Bewegungserfassungsplatte 1 umfasst eine Bodenplatte 14, welche unterhalb der Seitenteilplatten 12 und der Hauptteilplatte angeordnet ist. Zwischen der Bodenplatte 14 ist ein Zwischenraum 16 ausgebildet. In der Figur 3 ist die Bewegungserfassungsplatte 1 nur zu einem Teil gezeigt, was durch die gewellte Linie angedeutet ist. Im Zwischenraum 16 sind ein Kraftsensor 41, ein Vibrationsmotor 31, eine Kontrollvorrichtung 51, und ein Leuchtmittel 61 gezeigt. Der Kraftsensor 41 ist in Kontakt mit der Bodenplatte 14 und der Seitenteilplatte 12 und kann Druckverteilungen auf der Seitenteilplatte 12 erfassen. Der Vibrationsmotor 31 ist in Kontakt mit der Seitenteilplatte 12 angeordnet und kann haptische oder taktile Rückmeldungen auf die Seitenteilplatte übertragen. Die Kontrollvorrichtung 51 steuert die Kraftsensoren 41, die Vibrationsmotoren 31 und die Leuchtmittel 61. Die Leuchtmittel 61 umfassen LEDs, mittels welchen die Anzeigen 21 ausgeleuchtet werden können. Die Anzeigen 21 sind gestrichelt dargestellt und deuten transparente Teilflächen der Seitenteilplatten 12 an.

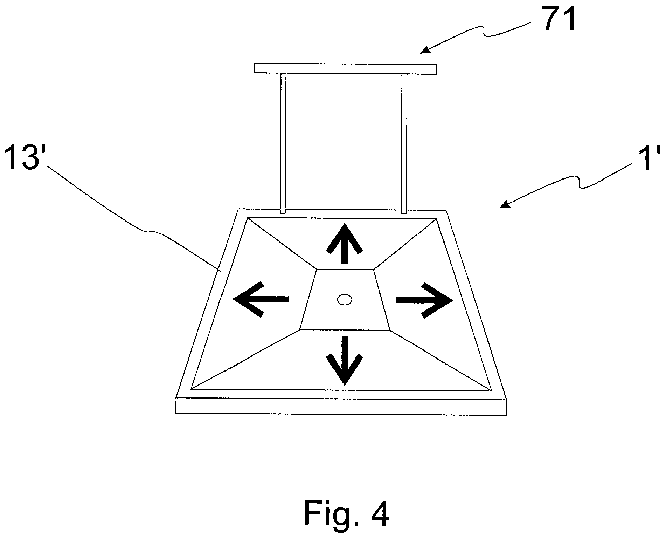

Figur 4 zeigt eine weitere Ausführungsform der Bewegungserfassungsplatte 1'. Die Bewegungserfassungsplatte 1' weist einen Rahmen 13' auf, an welcher eine Griffvorrichtung in Form eines Handlaufs 71 angebracht ist. Am Handlauf 71 kann sich eine auf der Bewegungserfassungsplatte positionierte Person gegebenenfalls festhalten. Am Handlauf 71 ist ein RFID-Reader (nicht gezeigt) angeordnet, über welche sich die Person für eine Trainingssession auf der Bewegungserfassungsplatte 1' identifizieren kann.

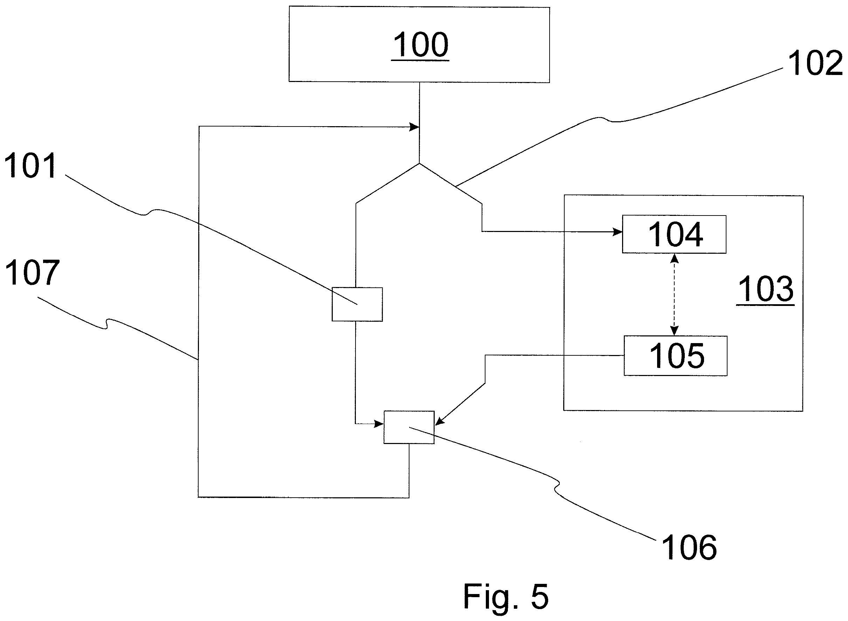

Figur 5 zeigt eine schematische Darstellung eines Ausführungsbeispiels des Verfahrens. Ein Satz an Stimulationssignalen mit einer vorgegebenen Komplexität wird am Anfang eines Zyklus von einer Datenbank 100 bereitgestellt. Der Satz an Stimulationssignalen wird einerseits in einer primären Kodiervorrichtung 101 in primäre Bewegungsdaten kodiert und andererseits in einen Verarbeitungskanal 102 geleitet. Der Verarbeitungskanal läuft in eine Störstelle 103, welcher eine Stimulationsvorrichtung 104 zur Ausgabe von visuellen Bewegungsanweisungen, welche aufgrund der Stimulationssignale generiert werden, und eine Bewegungserfassungsplatte 105 auf, auf welcher eine auf der Bewegungserfassungsplatte 105 positionierte Person sekundäre Bewegungsdaten erzeugt. Der gestrichelte Pfeil in der Störstelle 103 illustriert die Erzeugung der sekundären Bewegungsdaten auf der Bewegungserfassungsplatte 105 anhand der Bewegungsanweisungen aus der Stimulationsvorrichtung 104, welche vom Benutzer bedingt fehlerhaft, bzw. mit Abweichungen, stattfinden kann. Die sekundären Bewegungsdaten werden von der Störstelle ausgegeben und in einem Verarbeitungsmodul 106, z.B. einem Computer, mit den primären Bewegungsdaten aus der primären Kodiervorrichtung 101 verglichen. Im Verarbeitungsmodul 106 werden aus den Abweichungen der sekundären Bewegungsdaten von den primären Bewegungsdaten Differenzdaten gebildet. Als Funktion der Differenzdaten wird die Komplexität der Stimulationssignale verändert und als neuer Satz von Stimulationssignalen über den Rückführungskanal 107 ein einen neuen Zyklus gespiesen.

Der erfindungsgemäße Schutzumfang ist in den angefügten Ansprüchen definiert.

### LISTE DER BEZUGSZEICHEN

- 1,1': Bewegungserfassungsplatte
- 11: Hauptteilplatte
- 12: Seitenteilplatte
- 13, 13': Rahmen
- 14: Bodenplatte
- 16: Zwischenraum
- 21, 22: Anzeigevorrichtung
- 31: Vibrationsmotor
- 41: Kraftsensor
- 51: Kontrollvorrichtung
- 61: Leuchtmittel
- 71: Handlauf
- 100: Datenbank
- 101: Primäre Kodiervorrichtung
- 102: Verarbeitungskanal
- 103: Störstelle
- 104: Stimulationsvorrichtung
- 105: Bewegungserfassungsplatte
- 106: Verarbeitungsmodul
- 107: Rückführkanal

## Patentansprüche

1. Bewegungserfassungsplatte (1,1') zur Erfassung von Bewegungen einer auf der Bewegungserfassungsplatte (1,1') positionierten Person, umfassend mindestens drei Teilplatten (11, 12), welche eine gemeinsame Standfläche ausbildend angeordnet sind, wobei an jeder Teilplatte (11, 12) jeweils mindestens drei unabhängige Kraftsensoren (41) zur dynamischen Erfassung von lokalen Druckverteilungen angeordnet sind, wobei die Kraftsensoren (41) derart ausgebildet sind, dass die Teilplatten (11, 12) in Bezug auf Gewichtsänderungen eine Empfindlichkeit von 1 kg aufweisen, die Bewegungserfassungsplatte (1,1') weiter umfassend eine Rückmeldungsvorrichtung (21, 22, 31, 61) zur Erzeugung einer auditiven und/oder visuellen und/oder taktilen und/oder haptischen Rückmeldung und eine Kontrollvorrichtung (51) zur Ansteuerung der Kraftsensoren (41) und/oder der Rückmeldungsvorrichtung (21,22,31,61).

2. Bewegungserfassungsplatte (1,1') nach Patentanspruch 1, **dadurch gekennzeichnet, dass** die Rückmeldungsvorrichtung Vibrationsmotoren (31) zur Erzeugung einer haptischen oder taktilen Rückmeldung umfasst, wobei vorzugsweise an jeder Teilplatte (11, 12) jeweils mindestens ein durch die Kontrollvorrichtung (51) ansteuerbarer Vibrationsmotor (31) angeordnet ist.

3. Bewegungserfassungsplatte (1,1') nach Patentanspruch 1 oder 2, **dadurch gekennzeichnet, dass** die Rückmeldungsvorrichtung ausleuchtbare Anzeigevorrichtungen (21, 22) umfasst, welche an der Bewegungserfassungsplatte (1,1') angeordnet sind und durch die Kontrollvorrichtung (51) ansteuerbar sind.

4. Bewegungserfassungsplatte (1,1') nach einem der vorhergehenden Patentansprüche, **dadurch gekennzeichnet, dass** die Bewegungserfassungsplatte (1,1') eine Kommunikationsschnittstelle zur Ausgabe von durch die Kraftsensoren (41) erfassten Werten umfasst.

5. Bewegungserfassungsplatte (1,1') nach einem der vorhergehenden Patentansprüche, **dadurch gekennzeichnet, dass** die Bewegungserfassungsplatte (1,1') eine Bodenplatte (14) aufweist, über welche die Teilplatten (11 , 12) angeordnet sind, wobei die Kraftsensoren (41) zwischen der Bodenplatte (14) und den Teilplatten (11, 12) angeordnet sind.

6. Bewegungserfassungsplatte (1,1') nach einem der vorhergehenden Patentansprüche, **dadurch gekennzeichnet, dass** die Bewegungserfassungsplatte (1,1 ') fünf Teilplatten (11, 12) umfasst, wobei eine Teilplatte als zentral angeordnete Hauptteilplatte (11) ausgebildet ist und vier Teilplatten als Seitenteilplatten (12) um die zentrale Hauptteilplatte (11) herum angeordnet sind.

7. Bewegungserfassungsplatte (1,1') nach einem der vorhergehenden Patentansprüche, **dadurch gekennzeichnet, dass** an jeder Teilplatte (11, 12) vier Kraftsensoren (41) peripher angeordnet sind.

8. Bewegungserfassungsvorrichtung umfassend eine Bewegungserfassungsplatte (1,1') nach einem der Patentansprüche 1 bis 7 und eine Stimulationsvorrichtung (104) zur Ausgabe von auditiven und/oder visuellen Bewegungsanweisungen.

9. Bewegungserfassungsvorrichtung nach Patentanspruch 8, **dadurch gekennzeichnet, dass** die Bewegungserfassungsvorrichtung eine zur Identifikation einer Person ausgelegte Identifikationsvorrichtung mit einem RFID-Reader oder Barcode-Reader umfasst.

10. Verfahren zum Bereitstellen, dynamischen Erfassen und Verarbeiten von Stimulationssignalen und Bewegungsdaten einer Bewegungserfassungsvorrichtung nach Anspruch 8 oder 9, umfassend die Schritte:
a. Bereitstellen eines Satzes von Stimulationssignalen mit einer vorgegebenen Komplexität;
b. Leiten der Stimulationssignale in einen mit einer Störstelle (103) versehenen Verarbeitungskanal (102);
c. Bilden von primären Bewegungsdaten aus den Stimulationssignalen;
d. Herleiten von sekundären Bewegungsdaten aus den Stimulationssignalen in der Störstelle (103);
e. Ausgeben der sekundären Bewegungsdaten aus der Störstelle (103);
f. Vergleichen der sekundären Bewegungsdaten mit den primären Bewegungsdaten und Bilden von Differenzdaten;
g. Verändern der Komplexität des Satzes der Stimulationssignale als Funktion der Differenzdaten;
h. Bereitstellen eines neuen Satzes von Stimulationssignalen mit veränderter Komplexität
wobei das Herleiten der sekundären Bewegungsdaten in der Störstelle (103) folgende Schritte umfasst:
i. Erzeugen von räumlichen und/oder zeitlichen Bewegungsanweisungen mittels einer Stimulationsvorrichtung (104) anhand der Stimulationssignale;
j. Erfassen von sekundären Bewegungsdaten mittels einer Bewegungserfassungsplatte (1,1') gemäss einem der Patentansprüche 1 bis 7, wobei die sekundären Bewegungsdaten durch Bewegungen einer auf der Bewegungserfassungsplatte (1,1') positionierten Person gemäss den Bewegungsanweisungen erzeugt werden, wobei ein Korrekturzeitfenster vorgesehen ist, innerhalb welchem
die Person nach einer Sofortrückmeldung gegebenenfalls eine zu stark von den Bewegungsanweisungen abweichende Bewegung korrigieren kann.

11. Verfahren nach Patentanspruch 10, **dadurch gekennzeichnet, dass** nach der Erzeugung einer Bewegungsanweisung ein Zeitfenster definiert wird, wobei ein erstes in diesem Zeitfenster erfasstes sekundäres Bewegungsdatenelement einem der Bewegungsanweisung zugehörigen Stimulationssignal zugeordnet wird und die Zeit zwischen Anzeigen der Bewegungsanweisung und Erfassen des sekundären Bewegungsdatenelements als Reaktionszeitparameter in die Bildung eines Differenzdatenelements aufgenommen wird.

12. Verfahren nach Patentanspruch 10 oder 11, **dadurch gekennzeichnet, dass** die sekundären Bewegungsdaten aus den von den Kraftsensoren (41) der Bewegungserfassungsplatte (1,1') erfassten Druckverteilungswerten ermittelt werden.

13. Verfahren nach einem der Patentansprüche 10 bis 12, **dadurch gekennzeichnet, dass** bei oder ab einer vorgegebenen Differenz zwischen den sekundären Bewegungsdaten und den anhand der Stimulationssignale erzeugten Bewegungsanweisungen eine auditive und/oder visuelle und/oder haptische oder taktile Sofortrückmeldung durch die Rückmeldungsvorrichtung (21, 22, 31,61) ausgegeben wird.

14. Verfahren nach einem der Patentansprüche 10 bis 13, **dadurch gekennzeichnet, dass** die primären und/oder sekundären Bewegungsdaten und/oder Differenzdaten und/oder Stimulationssignale in einer Datenbank (100) gespeichert werden, wobei die Datenbank (100) vorzugsweise mit einer Managementoberfläche zur Bereitstellung eines Zugriffs auf die Datenbank (100) verbunden ist.

## Claims

1. Motion detection plate (1, 1') for detecting movements of a person positioned on the motion detection plate (1, 1'), comprising at least three partial plates (11, 12) which are arranged to form a common standing surface, at least three independent force sensors (41) being arranged on each partial plate (11, 12) for dynamically detecting local pressure distributions, the force sensors (41) being configured in such a way that the partial plates (11, 12) have a sensitivity of 1 kg with respect to weight changes, the motion detection plate (1, 1') further comprising a feedback device (21, 22, 31, 61) for generating an auditory and/or visual and/or tactile and/or haptic feedback and a control device (51) for controlling the force sensors (41) and/or the feedback device (21, 22, 31, 61).

2. Motion detection plate (1, 1') according to claim 1, **characterized in that** the feedback device comprises vibration motors (31) for generating a haptic or tactile feedback, wherein preferably at least one vibration motor (31) controllable by the control device (51) is arranged on each partial plate (11, 12).

3. Motion detection plate (1, 1') according to claim 1 or 2, **characterized in that** the feedback device comprises illuminable display devices (21, 22) which are arranged on the motion detection plate (1, 1') and can be controlled by the control device (51).

4. Motion detection plate (1, 1') according to one of the preceding claims, **characterized in that** the motion detection plate (1, 1') comprises a communication interface for outputting values detected by the force sensors (41).

5. Motion detection plate (1, 1') according to one of the preceding patent claims, **characterized in that** the motion detection plate (1, 1') has a base plate (14) via which the partial plates (11, 12) are arranged, the force sensors (41) being arranged between the base plate (14) and the partial plates (11, 12).

6. Motion detection plate (1, 1') according to one of the preceding claims, **characterized in that** the motion detection plate (1, 1') comprises five partial plates (11, 12), one partial plate being formed as a centrally arranged main partial plate (11) and four partial plates being arranged as side partial plates (12) around the central main partial plate (11).

7. Motion detection plate (1, 1') according to one of the preceding claims, **characterized in that** four force sensors (41) are arranged peripherally on each partial plate (11, 12).

8. Motion detection device comprising a motion detection plate (1, 1') according to one of the patent claims 1 to 7 and a stimulation device (104) for outputting auditory and/or visual motion instructions.

9. Motion detection device according to claim 8, **characterized in that** the motion detection device comprises an identification device configured to identify a person, comprising an RFID reader or bar code reader.

10. Method for providing, dynamically detecting and processing stimulation signals and motion data of a motion detection device according to Claim 8 or 9, comprising the steps of:
a. Providing a set of stimulation signals having a predetermined complexity;
b. Directing the stimulation signals into a processing channel (102) provided with an disturbance point (103);
c. Forming primary motion data from the stimulation signals;
d. Deriving secondary motion data from the stimulation signals in the disturbance point (103);
e. Outputting the secondary motion data from the disturbance point (103);
f. Comparing the secondary motion data with the primary motion data and forming difference data;
g. Changing the complexity of the set of stimulation signals as a function of the difference data;
h. Providing a new set of stimulation signals with modified complexity
wherein deriving the secondary motion data in the disturbance point (103) comprises the following steps
i. generating spatial and/or temporal motion instructions using a stimulation device (104) based on the stimulation signals;
j. detecting secondary motion data by means of a motion detection plate (1, 1') according to any one of claims 1 to 7, wherein the secondary motion data is generated by movements of a person positioned on the motion detection plate (1, 1') according to the motion instructions, wherein a correction time window is provided within which the person can correct a movement deviating too much from the motion instructions after an immediate feedback, if necessary.

11. Method according to patent claim 10, **characterized in that** a time window is defined after the generation of a motion instruction, wherein a first secondary motion data element detected in this time window is assigned to a stimulation signal associated with the motion instruction and the time between display of the motion instruction and detection of the secondary motion data element is included as a reaction time parameter in the generation of a difference data element.

12. Method according to claim 10 or 11, **characterized in that** the secondary motion data is determined from the pressure distribution values detected by the force sensors (41) of the motion detection plate (1, 1').

13. Method according to one of claims 10 to 12, **characterized in that** at or above a predetermined difference between the secondary movement data and the movement instructions generated on the basis of the stimulation signals, an auditory and/or visual and/or haptic or tactile immediate feedback is output by the feedback device (21, 22, 31, 61).

14. Method according to any one of claims 10 to 13, **characterized in that** the primary and/or secondary motion data and/or difference data and/or stimulation signals are stored in a database (100), the database (100) preferably being connected to a management interface for providing access to the database (100).

## Revendications

1. Plaque de détection de mouvement (1, 1') pour la détection de mouvements d'une personne positionnée sur la plaque de détection de mouvement (1, 1'), comprenant au moins trois plaques partielles (11, 12) qui sont disposées de manière à former une surface d'appui commune, au moins trois capteurs de force (41) indépendants étant respectivement disposés sur chaque plaque partielle (11, 12) pour la détection dynamique de distribution de pression locales, les capteurs de force (41) étant conçus de telle sorte, que les plaques partielles (11, 12) présentent une sensibilité de 1 kg par rapport aux variations de poids, la plaque de détection de mouvement (1, 1') comprend en outre un dispositif de rétroaction (21, 22, 31, 61) pour générer une rétroaction auditive et/ou visuelle et/ou tactile et/ou haptique et un dispositif de contrôle (51) pour commander les capteurs de force (41) et/ou le dispositif de rétroaction (21, 22, 31, 61).

2. Plaque de détection de mouvement (1, 1') selon la revendication 1, **caractérisée en ce que** le dispositif de rétroaction comprend des moteurs vibrants (31) pour produire une rétroaction haptique ou tactile, au moins un moteur vibrant (31) pouvant être commandé par le dispositif de contrôle (51) étant de préférence disposé sur chaque plaque partielle (11, 12).

3. Plaque de détection de mouvement (1, 1') selon la revendication 1 ou 2, **caractérisée en ce que** le dispositif de rétroaction comprend des dispositifs d'affichage (21, 22) pouvant être éclairés, qui sont disposés sur la plaque de détection de mouvement (1, 1') et qui peuvent être commandés par le dispositif de contrôle (51).

4. Plaque de détection de mouvement (1, 1') selon l'une des revendications précédentes, **caractérisée en ce que** la plaque de détection de mouvement (1, 1') comprend une interface de communication pour la sortie de valeurs détectées par les capteurs de force (41).

5. Plaque de détection de mouvement (1, 1') selon l'une des revendications précédentes, **caractérisée en ce que** la plaque de détection de mouvement (1, 1') présente une plaque de fond (14) au-dessus de laquelle sont disposées les plaques partielles (11, 12), les capteurs de force (41) étant disposés entre la plaque de fond (14) et les plaques partielles (11, 12).

6. Plaque de détection de mouvement (1, 1') selon l'une des revendications précédentes, **caractérisée en ce que** la plaque de détection de mouvement (1, 1') comprend cinq plaques partielles (11, 12), une plaque partielle étant conçue comme plaque partielle principale (11) disposée de manière centrale et quatre plaques partielles étant disposées comme plaques partielles latérales (12) autour de la plaque partielle principale centrale (11).

7. Plaque de détection de mouvement (1, 1') selon l'une des revendications précédentes, **caractérisée en ce que** quatre capteurs de force (41) sont disposés à la périphérie de chaque plaque partielle (11, 12).

8. Dispositif de détection de mouvement comprenant une plaque de détection de mouvement (1, 1') selon l'une des revendications 1 à 7 et un dispositif de stimulation (104) pour émettre des instructions de mouvement auditives et/ou visuelles.

9. Dispositif de détection de mouvement selon la revendication 8, **caractérisé en ce que** le dispositif de détection de mouvement comprend un dispositif d'identification conçu pour identifier une personne et comprenant un lecteur RFID ou un lecteur de code à barres.

10. Procédé de mise à disposition, d'acquisition dynamique et de traitement de signaux de stimulation et de données de mouvement d'un dispositif de détection de mouvement selon la revendication 8 ou 9, comprenant les étapes suivantes :
a. Fournir un ensemble de signaux de stimulation d'une complexité prédéterminée ;
b. diriger les signaux de stimulation vers un canal de traitement (102) pourvu d'un point de perturbation (103) ;
c. Formation de données de mouvement primaires à partir des signaux de stimulation ;
d. Dérivation des données de mouvement secondaires à partir des signaux de stimulation dans le point de perturbation (103) ;
e. Édition des données de mouvement secondaires à partir du point de perturbation (103) ;
f. Comparaison des données de mouvement secondaires avec les données de mouvement primaires et formation de données de différence ;
g. Modifier la complexité de l'ensemble des signaux de stimulation en fonction des données différentielles ;
h. Fournir un nouvel ensemble de signaux de stimulation avec une complexité modifiée
dans lequel la dérivation des données de mouvement secondaires dans le point de perturbation (103) comprend les étapes suivantes :
i. la génération d'instructions de mouvement spatiales et/ou temporelles au moyen d'un dispositif de stimulation (104) à l'aide des signaux de stimulation ;
j. Saisie de données de mouvement secondaires au moyen d'une plaque de saisie de mouvement (1, 1') selon l'une des revendications 1 à 7, les données de mouvement secondaires étant générées par des mouvements d'une personne positionnée sur la plaque de saisie de mouvement (1, 1') selon les instructions de mouvement, une fenêtre de temps de correction étant prévue, à l'intérieur de laquelle la personne peut éventuellement corriger un mouvement s'écartant trop fortement des instructions de mouvement après une confirmation immédiate.

11. Procédé selon la revendication 10, **caractérisé en ce qu'**une fenêtre temporelle est définie après la génération d'une instruction de mouvement, un premier élément de données de mouvement secondaire détecté dans cette fenêtre temporelle étant associé à un signal de stimulation correspondant à l'instruction de mouvement, et le temps entre l'affichage de l'instruction de mouvement et la détection de l'élément de données de mouvement secondaire étant inclus en tant que paramètre de temps de réaction dans la génération d'un élément de données de différence.

12. Procédé selon la revendication 10 ou 11, **caractérisé en ce que** les données de mouvement secondaires sont déterminées à partir des valeurs de distribution de pression détectées par les capteurs de force (41) de la plaque de détection de mouvement (1, 1').

13. Procédé selon l'une des revendications 10 à 12, **caractérisé en ce qu'**à ou à partir d'une différence prédéterminée entre les données de mouvement secondaires et les instructions de mouvement générées à l'aide des signaux de stimulation, une rétroaction immédiate auditive et/ou visuelle et/ou haptique ou tactile est émise par le dispositif de rétroaction (21, 22, 31, 61).

14. Procédé selon l'une quelconque des revendications 10 à 13, **caractérisé en ce que** les données de mouvement primaires et/ou secondaires et/ou les données différentielles et/ou les signaux de stimulation sont stockés dans une base de données (100), la base de données (100) étant de préférence associée à une interface de gestion pour fournir un accès à la base de données (100).
